# EUROPEAN PATENT APPLICATION

(11) **EP 1 808 413 A1**
(43) Date of publication of application: **18.07.2007**
(21) Application number: 05781414.7
(22) Date of filing: 02.09.2005
(51) Int. Cl.: C01G 49/00, A61F 7/00

(54) **POWDER MATERIAL GENERATING HEAT IN AC MAGNETIC FIELD AND METHOD FOR PRODUCING SAME**

(30) Priority: 16.09.2004 JP 2004270013
(71) Applicant: Ad Me Tech Co., Ltd., Matsuyama-shi Ehime 7918042 (JP)
(72) Inventor: AONO, Hiromichi, 7918005 (JP); MAEHARA, Tsunehiro, 7910104 (JP); HATTORI, Yasumasa SUMITOMO METALMINING CO., LTD, Niihama-shi Ehime 7920002 (JP); MORI, Kensaku SUMITOMO METALMINING CO., LTD, Niihama-shi Ehime 7920002 (JP)
(74) Representative: Morelle, Guy Georges Alain
(86) International application number: PCT/JP2005/016149
(87) International publication number: WO 2006/030655

(57) **Abstract**

This invention provides a powder material, which is capable of obtaining a heating value that is greater than that of MgFe₂O₄, and the manufacturing method thereof.

The powder material is expressed by the general formula Mg₁₋ₓAₓFe₂O₄ (where A in the formula is at least one element selected from the group of Ca, Sr and Ba, and the range of x in the formula is 0.05 ≤ x ≤ 0.95). It is preferred that the range of x in the general formula be 0.2 ≤ x ≤ 0.8. Also, it is preferred that the size of crystallites on the (440) surface be 80 to 200Å.

## Description

### BACKGROUND OF THE INVENTION

The present invention relates to a powder material that generates heat in an alternating current magnetic field and the manufacturing method thereof, and more particular to powder material having excellent heat generating characteristics in an alternating current magnetic field and the manufacturing method thereof that can be used in hyperthermia treatment such as that performed in the treatment of cancer.

As one method for treating cancer is a method called hyperthermia. This is a method in which an affected area is heated by applying a magnetic field having an alternating current of about 8 MHz from the outside of the body of a cancer patient. Normal cells are also heated, however, the normal cells are heated to only about 40°C (degree centigrade), whereas, since the blood flow in the tumor area is poor, the tumor area is heated to about 42°C. In this way it is possible to apply damage to the cancer cells of the affected area.

However, since the temperature during heating is a low 42°C, it is currently difficult for the effects of that treatment to be recognized, and in order to improve the effects, it is necessary to heat the affected area to a higher temperature.

Therefore, a method has been tested as a method for treating cancer using an alternating current magnetic field in which a powder material that generates heat in an alternating current magnetic field is administered to the tumor area, and by applying a high-frequency magnetic field, the tumor is coagulated and cauterized. Magnetite (FeFe₂O₄) is typically used as the powder material in this case. However, there is a problem with chemical stability in that the bivalent iron (Fe⁺²) in the FeFe₂O₄ structure is easily oxidized to trivalent iron (Fe⁺³).

An example of material for which this problem has been solved is Magnesium Ferrite (MgFe₂O₄) that has been reported by the inventors of the present invention (see patent document 1, non-patent document 1). The magnesium is not oxidized to bivalent magnesium or greater, so MgFe₂O₄ is chemically stable, and at a frequency of about 400 kHz it is possible to obtain a large heat generating value of 100°C or greater.

Here, excellent heat generation property per unit mass is desired as a property of the material used for hyperthermia treatment. The reason for this is that when considering administration to inside a cell, it is best that the amount administered be as small as possible. Therefore, it is desired that a material capable of obtaining a larger heating value than MgFe₂O₄ be developed.

There is a close relationship between the particle size and the heat generation properties of a magnetic body as described above.

However, in the solid-phase reaction of a raw material such as a typically used oxide, it is necessary to perform calcination at a high temperature, so particles are formed at a larger size than necessary. Therefore, it is difficult to manufacture a material while controlling the particle size so that it has the best heat generation properties.
[Patent Document 1] Japanese Patent Application Publication No. 2004-89704
[Non-patent Document 1] Jpn. J. Appl. Phys., Vol. 41(3), 1620-1621 (2002)

### SUMMARY OF THE INVENTION

Taking the aforementioned problem into consideration, the object of the present invention is to provide a powder material, which is capable of a heating value that is larger than that of MgFe₂O₄, and the manufacturing method thereof.

The powder material of the present invention is expressed by the general formula Mg₁₋ₓAₓFe₂O₄ (where A in the formula is at least one element selected from the group of Ca, Sr and Ba, and the range of x in the formula is 0.05 ≦ x ≦ 0.95).

It is preferred that the range of x in the general formula be 0.2 ≦ x ≦ 0.8.

It is preferred that the size of crystallites on the (440) surface be 80 to 200Å (Angstrom). Here, the size of the crystallites on the (440) surface is found by performing X-ray diffraction measurement on powder test samples, and calculating the size according to the Scherrer method.

The method for manufacturing the powder material of this invention is a method for manufacturing a powder material in which the powder is expressed by the general formula Mg₁₋ₓAₓFe₂O₄ (where A in the formula is at least one element selected from the group of Ca, Sr and Ba, and the range of x in the formula is 0.05 ≦ x ≦ 0.95), and comprising: a process of coprecipitating out a hydroxide of Mg, element A and Fe from an aqueous solution in which Mg, element A and Fe are dissolved; and a process of performing calcination at a temperature of 700 to 900°C on the coprecipitated hydroxide.

### EFFECT OF THE INVENTION

The powder material of this invention has better heat generation properties in an alternating current magnetic field than prior powder materials, so by using the powder material of the present invention in hyperthermia treatment, it is possible to obtain sufficient heat generation, as well as it is possible to reduce the amount of material that is administered into the cells, which makes it possible to decrease the burden on the patient.

### BRIEF DESCRIPTION OF THE DRAWINGS

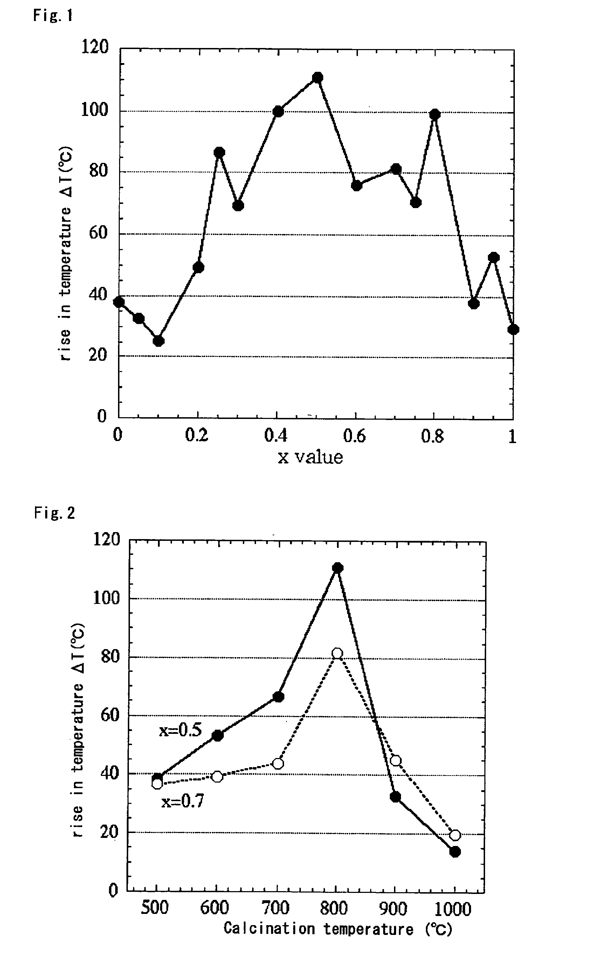

Fig. 1 shows the rise in temperature ΔT of 1g test samples of powder, for which the value of the relative proportion x of Ca in a powder having the composition Mg₁₋ₓCaₓFe₂O₄ is changed to specified values within the range from 0 to 1, when maintained in an alternating current magnetic field (frequency: 370 kHz, magnetic field strength: 4 kA/m) for 20 minutes.
FIG. 2 shows the temperature rise ΔT of 1g of each powder material Mg₁₋ₓCaₓFe₂O₄, which are obtained by performing calcination for one hour of the precursor (test samples of Mg₁₋ₓCaₓFe₂O₄ for which x = 0.5, and x = 0.7) at the temperatures 500°C, 600°C, 700°C, 800°C, 900°C and 1000°C, when maintained in an alternating current magnetic field (frequency: 370 kHz, Magnetic field strength: 4 kA/m) for 20 minutes.

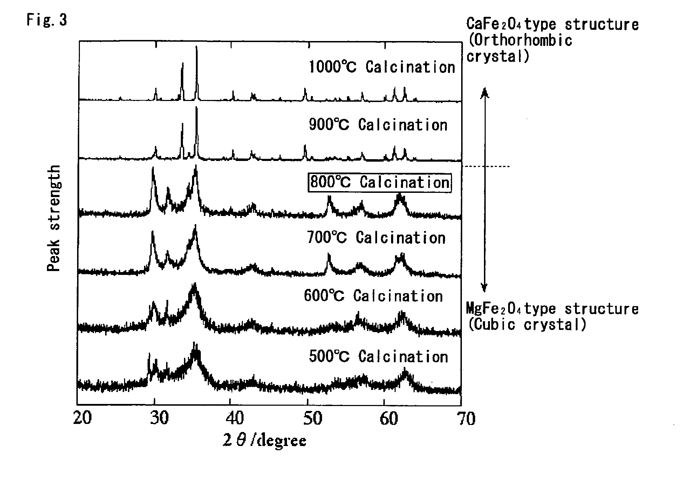

FIG. 3 shows the result of X-ray diffraction of the powder Mg_{0.3}Ca_{0.7}Fe₂O₄ of which the relative proportion of Ca is 0.7 after calcination has been performed for one hour at the temperatures 500°C, 600°C, 700°C, 800°C, 900°C and 1000°C.

### DESCRIPTION OF THE PREFERRED EMBODIMENTS

Ferrite is a material having mainly Ferrimagnetic properties, and is a material that generates heat in an alternating current magnetic field when loss such as hysteresis loss changes to heat. Also, the inventors have already proposed MgFe₂O₄ as a powder material having excellent heat generation properties in an alternating current magnetic field (see non-patent document 1).

In order to further build on this research result, the inventors have continued performing research and development in order to further improve the heat generation properties.

As a result, it was found that the heat generation properties could be improved by replacing a specified amount of Mg in the MgFe₂O₄ with Ca, which has a larger ion radius than Mg. It was also found that the heat generation properties could be even further improved by performing calcination of Mg₁₋ₓCaₓFe₂O₄, of which a specified amount of Mg has been replaced with Ca, at a temperature near the temperature at which transition occurs from a MgFe₂O₄ type cubic crystal to a CaFe₂O₄ type orthorhombic crystal, or in other words at a temperature near 800°C.

The present invention was accomplished based on this prior finding.

The reasons for numerical limitations and the like for each of the compositional requirements for the powder material and manufacturing method thereof of this invention are explained below.

### [General Formula: (A in the formula is at least one element that is selected from the group of Ca, Sr and Ba, and the range of x in the formula is 0.05 ≤ x ≤ 0.95)]

The value x in the formula indicates the relative proportion of element A that replaces Mg. Here, the element A is an element selected from the group of Ca, Sr and Ba. When the value of x is less than 0.05 or greater than 0.95, the effect of replacing Mg with the element A is not obtained.

When the range of x in the formula is 0.2 ≤ x ≤ 0.8, the heat generation properties become even better.

The reason it is possible to improve the heat generation properties by replacing Mg with the element A is that the ion radius of the element A is greater than that of Mg, so by replacing Mg with element A, strain is introduced into the crystal, which is thought to be due to an increase in hysteresis loss and an increase in the heating value.

The element A is selected from at least one element from the group of Ca, Sr and Ba, however, when taking into consideration introduction into the body, Ca is preferred.

Furthermore, it is preferred that the size of the crystallites on the surface (440) of the powder material that is expressed by the general formula Mg₁₋ₓAₓFe₂O₄ be 80 to 200Å. The calcination temperature when the size of the crystallites of the surface (440) becomes 80 to 200Å is near the temperature at which the crystal structure of the powder material transitions from a MgFe₂O₄ type cubic crystal to a CaFe₂O₄ type of orthorhombic crystal, or in other words near 800°C, so strain is further introduced into the crystal by calcination, which is due to the further increase in hysteresis loss and increase in heating value.

Element A is at least one element selected from the group of Ca, Sr and Ba, and since similar to Mg, the bivalent positive ion state of Ca, Sr and Ba is stable, the crystal that is expressed by the general formula Mg₁₋ₓAₓFe₂O₄ is chemically stable.

### Method for Manufacturing the Powder Material of the Invention

In order to manufacture the powder material of the present invention, first, magnesium salt such as MgCl₂ • 6H₂O, calcium salt such as CaCl₂, and Fe(III) salt such as FeCl₃ • 6H₂O, for example, are dissolved in water, then Mg, Ca and Fe are coprecipitated as hydroxide by the addition of alkali. In this way, the precursor of the powder material is obtained. Then by filtering and performing calcination of the precursor at a specified temperature, it is possible to manufacture the powder material of the present invention.

Here, by making the calcination temperature near the temperature at which the crystal structure of the powder material transitions from a cubic crystal to an orthorhombic crystal, or in other words, a temperature of 700 to 900°C, it is possible to introduce strain into the crystals and to bring about miniaturization of the crystals, which makes it possible to make the heat generation properties of the powder material better. The calcination temperature is a relatively low temperature of 1000°C or less, and even though calcination is performed at this kind of temperature, growth and accumulation of crystal grains does not occur much. Therefore, by pulverization it is easy to obtain fine grains having a grain size of 0.05 to 0.2 µm. In order to eliminate accumulation due to calcination, or in order to perform pulverization, it is possible to use a pulverizing apparatus such as a ball mill or bead mill.

### Embodiments of the Powder Material of the Invention

When using the powder material Mg₁₋ₓAₓFe₂O₄ of the present invention in hyperthermia treatment, it is preferred from the aspect of reducing the amount of material that is used in hyperthermia treatment that the material be made from pure ferrite having the composition Mg₁₋ₓAₓFe₂O₄, however, it is also possible for the material to be a mixture that contains a high proportion of unreacted material or side reaction material. Also, it is possible to mix a cementing material such as organic matter to the powder material and form the powder material into a specified shape such as an acicular shape, then coat the powder material with organic matter in order to improve the affinity for the body.

### Embodiment

The powder material Mg₁₋ₓCaₓFe₂O₄ was manufactured as described below. In order that x of the obtained material was a specified value within the range 0 to 1, MgCl₂ • 6H₂O (Kanto Chemical Co., Inc.), CaCl₂ (Kanto Chemical Co., Inc) and FeCl₃ • 6H₂O (Kanto Chemical Co., Inc) were dissolved at a stoichiometric mixture ratio to create an aqueous solution. This aqueous solution was dripped into 6 regulated NaOH aqueous solutions that were heated at 100°C, and the precursor was precipitated out as a hydroxide. This was filtered and the obtained hydroxide precursor was calcinated for 1 hour at 800°C, and each powder materials, having the Mg₁₋ₓCaₓFe₂O₄ composition of which the value of x that indicates the relative proportion of Ca was changed to specified values within the range 0 to 1, were manufactured.

One gram of each of the test powders was placed in an alternating current magnetic field (frequency: 370 kHz, magnetic field strength: 4 kA/m) for 20 minutes. The rise in temperature ΔT of the test samples for this case are shown in FIG. 1. The rise in temperature ΔT is the result obtained by subtracting the temperature of the test sample at the beginning of the test from the temperature of the test sample after being kept in an alternating current magnetic field (frequency: 370 kHz, magnetic field strength: 4 kA/m) for 20 minutes.

As can be seen from FIG. 1, compared with the rise in temperature of the material MgFe₂O₄ having a composition in which x = 0, the temperature rise of the material increased more the larger the value of x that indicates the relative proportion of Ca was within the range.

After obtaining the hydroxide precursors (test samples for which the values of x in Mg₁₋ₓCaₓFe₂O₄ are x = 0.5, and x = 0.7) as described above, these precursors were each calcinated for 1 hour at the temperatures 500°C, 600°C, 700°C, 800°C, 900°C and 1000°C, to obtain test samples in order to check the effect of the heat processing temperature. One gram of each of the obtained test samples was placed in an alternating current magnetic field (frequency: 370 kHz, magnetic field strength: 4 kA/m) for 20 minutes. The rise in temperature ΔT of the test samples for this case are shown in FIG. 2. The rise in temperature ΔT is the result obtained by subtracting the temperature of the test sample at the beginning of the test from the temperature of the test sample after being kept in an alternating current magnetic field (frequency: 370 kHz, magnetic field strength: 4 kA/m) for 20 minutes.

As can be seen in FIG. 2, the highest heat generation was obtained when calcination was performed at 800°C.

Next, in order to investigate the effect of heat processing temperature, X-ray diffraction measurement was performed on each powder of the aforementioned test samples (Mg_{0.3}Ca_{0.7}Fe₂O₄, for which the relative proportion of Ca is 0.7).

FIG. 3 shows the X-ray diffraction results. It can be seen that the peak changes bordering on the temperature of 800°C, and that the crystal structure changes. At 800°C or less, the powder has a MgFe₂O₄ type cubic crystal structure, and at 900°C or greater, the powder has a CaFe₂O₄ type orthorhombic crystal structure.

In the result of powder X-ray diffraction of Mg_{0.3}Ca_{0.7}Fe₂O₄ for which calcination was performed at 800 °C , the peak becomes rather irregular, and it can be seen that phase transition is occurring. This is thought to be the cause of the strain occurring in the crystal. Also, in the result of powder X-ray diffraction of Mg_{0.3}Ca_{0.7}Fe₂O₄ for which calcination was performed at below 800°C, the peak becomes rather broad, so the Mg_{0.3}Ca_{0.7}Fe₂O₄ powder material for which calcination was performed at below 800°C can be considered to be a very fine particle material having small crystallites.

In the case of Mg_{0.3}Ca_{0.7}Fe₂O₄ for which calcination was performed at 800°C in this way, strain is introduced into the crystal due to calcination, and the crystallites become very fine. Therefore, as shown in FIG. 2, it is thought that the highest amount of heat generation will be obtained when calcination is performed at 800°C.

Furthermore, the rise in temperature ΔT was measured in the same way for each of the test samples that were obtained by performing calcination for 1 hour at the temperatures 500°C, 700°C and 800°C on the precursors for which x in the formula Mg₁₋ₓCaₓFe₂O₄ is x = 0.3, 0.5, and 0.7, and powder X-ray diffraction measurement was performed, after which the size of the crystallites of the (440) surface was analyzed from the results using the Scherrer method. Those results are shown in Table 1.

**Table 1**

| Composition | Mg_{0.7} | Ca_{0.3} | Fe₂O₄ | Mg_{0.5} | Ca_{0.5} | Fe₂O₄ | Mg_{0.3}Ca_{0.7} | Fe₂O₄ |
|---|---|---|---|---|---|---|---|---|
| Sintering Temperature (°C) | 800 | 700 | 500 | 800 | 700 | 500 | 800 | 700 |
| ΔT(°C) | 81 | 44 | 37 | 110 | 66 | 38 | 80 | 42 |
| Crystallite Size(Å) | 188 | 104 | 58 | 131 | 100 | 61 | 106 | 97 |

As can be seen from Table 1, when the size of the crystallites on the (440) surface is 80Å or greater, the temperature rise ΔT exceeds 40°C, which can be seen as a good heat generation property.

### Industrial Application

The powder material of this invention has better heat generation properties in an alternating current magnetic field than prior powder materials, so it is possible to reduce the amount of material that is administered into the cells, which makes it possible to decrease the burden on the patient, and thus this powder material is suitable for use in hyperthermia treatment such as in cancer treatment.

## Claims

1. A powder material that is expressed by the general formula Mg₁₋ₓAₓFe₂O₄ (where A in the formula is at least one element selected from the group of Ca, Sr and Ba, and the range of x in the formula is 0.05 ≤ x ≤ 0.95).

2. The powder material of claim 1 wherein the range of x in the general formula is 0.2 ≤ x ≤ 0.8.

3. The powder material of claim 2 wherein the size of crystallites on the (440) surface is 80 to 200Å.

4. A method for manufacturing a powder material in which the powder is expressed by the general formula Mg₁₋ₓAₓFe₂O₄ (where A in the formula is at least one element selected from the group of Ca, Sr and Ba, and the range of x in the formula is 0.05 ≤ x ≤ 0.95), and comprising:
a process of coprecipitating out a hydroxide of Mg, element A and Fe from an aqueous solution in which Mg, element A and Fe are dissolved; and
a process of performing calcination at a temperature of 700 to 900°C on the coprecipitated hydroxide.

5. The method for manufacturing a powder material of claim 4 wherein the powder material is manufactured so that the range of x in the general formula is 0.2 ≤ x ≤ 0.8.

## Amended claims

### Amended claims under Art. 19.1 PCT

1. (Amended) A powder material having an excellent heat generation property in an alternative current magnetic field that is expressed by the general formula Mg₁₋ₓCaₓFe₂O₄ (where the range of x in the formula is 0.05 ≤ x ≤ 0.95).

2. (Amended) The powder material having an excellent heat generation property in an alternative current magnetic field of claim 1, wherein the range of x in the general formula is 0.35 < x ≤ 0.8.

3. (Amended) The powder material having an excellent heat generation property in an alternative current magnetic field of claim 1 or 2, wherein the size of crystallites on the (440) surface is 80 to 200 Å.

4. (Amended) A method for manufacturing a powder material having an excellent heat generation property in an alternative current magnetic field in which the powder is expressed by the general formula Mg₁₋ₓCaₓFe₂O₄ (where the range of x in the formula is 0.05 ≤ x ≤ 0.95), and comprising:
a process of coprecipitating out a hydroxide of Mg, Ca and Fe from an aqueous solution in which Mg, Ca and Fe are dissolved; and
a process of performing calcination at a temperature of 700 to 900 °C on the coprecipitated hydroxide so that the crystal of the powder material is strained.

5. (Amended) The method for manufacturing a powder material having an excellent heat generation property in an alternative current magnetic field of claim 4, wherein the powder material is manufactured so that the range of x in the general formula is 0.3 5 < x ≤ 0.8.

Statement under Art. 19.1 PCT
In claims 1- 5, the applicant describes that the powder material of this invention has "excellent heart generating characteristics in an alternating current magnetic field" and makes it clear that the powder material related to this invention is suitable for use as the material for heat generation. On the other hand, the powder material described in cited reference is not intended to be used as the material for hear generation.

In claim 4, the applicant makes it clear that the manufacturing method for the powder material of this invention includes the process that "strain is introduced into the crystal by performing calcinations at a temperature of 700 to 900 °C", and this process enhances the excellent heart generating characteristics of the powder material further.

The powder material described in cited reference 1 is performed calcinations at 1150 °C, and also the manufacturing method described in cited reference 2 does not relate to the heart generating characteristics. Therefore, there is no indication in either reference that adapts said manufacturing method in order to give heart generating characteristic to the material described in cited references.

In the claims 2 and 5, by amending the range of x is "0.35 < x < 0.8", the applicant makes it clear that the composition described in the cited reference 1 is not included in the preferred embodiments of the claimed invention.
